# NEW EUROPEAN PATENT SPECIFICATION

(11) **EP 0 598 250 B2**
(45) Date of publication and mention of the opposition decision: **26.01.2005**
(45) Mention of the grant of the patent: 04.09.1996
(21) Application number: 93117226.6
(22) Date of filing: 25.10.1993
(51) Int. Cl.: C07C 273/04

(54) **Method of retrofitting a pre-existing plant for urea production including an ammonia stripping section**
Verfahren zur Nachrüstung einer bestehenden, einen Ammoniak-Stripper enthaltenden Anlage zur Produktion von Harnstoff
Méthode pour adapter une usine, déjà exsistante pour la production d'urée qui contient une section de strippage d'ammoniaque

(30) Priority: 19.11.1992 CH 354492
(43) Date of publication of application: 25.05.1994
(73) Proprietor: UREA CASALE S.A., 6900 Lugano-Besso (CH)
(72) Inventor: Pagani, Giorgio, I-20142 Milano (IT); Zardi, Umberto, CH-6932 Breganzona (CH)
(74) Representative: Zardi, Marco

(56) References cited:
- EP-A- 0 155 735
- EP-A- 0 479 103
- EP-A- 0 497 215
- GB-A- 1 147 734
- US-A- 3 091 637
- US-A- 4 210 600
- Dr. Granelli, Schematic results of calculation
- English translation of NL 6808167A
- R: Powell, "Urea Process Technology", Noyes Dev. Corp., 1968, pp. 88-93 and 143-146

## Description

The present invention relates to a method of retrofitting a pre-existing plant for urea production including a first urea synthesis reactor in fluid communication with an ammonia stripping section for separating free ammonia and carbamate leaving the reactor from an aqueous urea solution, which comprises the step of providing a second urea synthesis reactor of the "once through" type having a higher efficiency yield than said first urea synthesis reactor.

As is well known, the need often arise of increasing the yield and urea production capacity of a pre-existing plant.

To this end, various methods of enhancing the production capacity by retrofitting a pre-existing plant, have been proposed heretofore, such as that described in European Patent application EP-A-0 479 103 by the same applicant.

EP-A-0 479103 discloses a urea production process wherein highly pure ammonia and carbon dioxide are reacted in a first reaction space, the reaction mixture thus obtained is fed to a recovery section and a synthesis reaction between less pure reagents, substantially recyded by the recovery section (recovery mixture), is carried out in a second reaction space.

According to the above-mentioned European Patent application, a capacity increase of a pre-existing plant is achieved by adding a "once through" high-yield reactor (i.e. fed by pure reagents without recycle water) in parallel to a pre-existing one, which operates in accordance to the total recycle technology (i.e. fed not only by pure reagents, but also with an aqueous carbamate recycle solution).

Even though a urea production capacity increase may accomplished in this way, a number of drawbacks still hinder an effective retrofitting of urea production plants including an ammonia stripping section.

In accordance with EP-A-0 479 103, in fact, the reaction mixture leaving the added reactor cannot be treated in the ammonia stripping section but must by-pass the same with the consequent need of a capacity increase of the recovery section downstream of the two synthesis parallel reactors, and with a substantial increase of the overall energy consumption of the urea synthesis process.

The problem underlying the present invention is therefore that of providing a method of retrofitting a urea production plant which allows a substantial capacity increase, while reducing at the same time the overall energy consumption required by the retrofitted plant thus obtained.

This problem is solved, according to the invention, by a method of retrofitting as indicated in attached claim 1 and which is characterized in that it comprises the steps of:
a) connecting said second reactor to and upstream of said ammonia stripping section and with means for feeding high purity ammonia and carbon dioxide;
b) distributing overall production capacity to apportion from 50 to 95% of said capacity to said first urea synthesis reactor and from 5 to 50% to said second urea synthesis reactor
c) reducing the production capacity of the pre-existing reactor.

In accordance with a first advantageous aspect of the present invention, the required capacity increase and reduction of energy consumption are simultaneously achieved by reducing the load of the pre-existing reactor and by feeding the added reactor so as to reach with the latter the new, higher, value of production capacity.

Most advantageously, the added reactor is of the "once through" type with partial removal of the reaction heat (non-adiabatic): the overall yield of the retrofitted plant may also be simultaneously enhanced in this way.

Further aspects and advantages of the method according to the present invention will be better apparent from the following description of a non-limitative preferred embodiment thereof, given by way of illustration with reference to the attached drawings, in which:
- Fig. 1 is a conventional schematic layout of a urea production plant including an ammonia stripping section according to the prior art:
- Fig. 2 is a schematic layout representing a plant obtainable by retrofitting the plant shown in Fig. 1 in accordance with the present invention; and
Fig. 3 is a schematic layout of the plant shown in Fig. 2 with a non-adiabatic "once through" reactor.

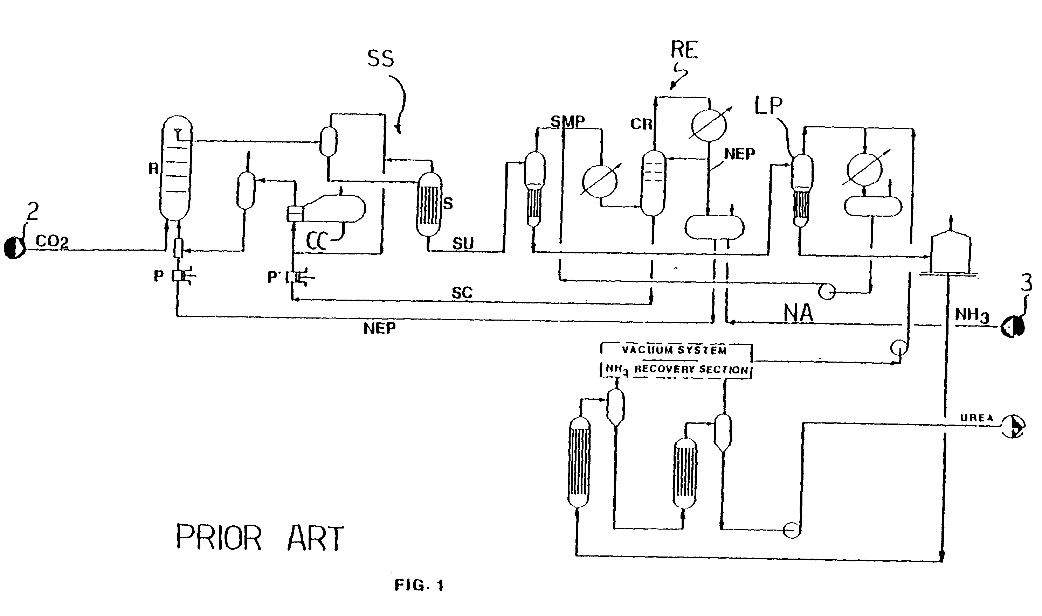

In Fig. 1, wherein a plant according to the prior art is shown, R indicates a urea synthesis reactor, fed by conventional means 2, 3 with pure carbon dioxide and ammonia respectively, which is in fluid communication with an ammonia stripping section SS provided with a stripper S.

In the stripper S a large part of the carbamate contained in the urea solution leaving the reactor R and part of the free ammonia present are stripped and recycled to the reactor, while a urea solution SU is obtained having a relatively low residue carbon dioxide content (5ö7% by weight), and a relatively high ammonia content (22ö25% by weight).

The stripping section SS is in turn in fluid communication with a urea recovery section RE, including a medium pressure distillation stage SMP, operating at 18ö20 bar, a rectifying column CR and a low pressure distiller LP operating at about 4 bar.

In the rectifying column CR, a highly pure ammonia stream NEP is separated from a carbamate solution SC.

Pump means P, P' are respectively provided for feeding pure ammonia, obtained by mixing the ammonia stream NEP with a fresh stream NA, and for recycling the carbamate solution SC to a carbamate condenser CC and to the synthesis reactor R.

The main technical characteristics of the isobaric ammonia stripping process implemented by the aforementioned plant can be summarized as follows:

| | |
|---|---|
| - synthesis pressure | 140 - 150 bar |
| - NH3/CO2 mol in the reactor | 3.2ö3.4 |
| - H20/CO2 mol in the reactor | 0.6ö0.8 |
| - temperature of the reactor | 180 - 190°C |
| - yield | 62ö63% |
| - steam consumption | about 900 kg/MT urea |

The above mentioned values were quite consolidated and great improvements to the process appeared not to be possible.

According to the present invention, the plant shown in Fig. 1 is retrofitted by:
i) providing, upstream of the ammonia stripping section SS, a second urea synthesis reactor ROT of the "once through" type having a higher efficiency yield than the first urea synthesis reactor R;
ii) connecting reactor ROT with the ammonia stnpping section SS by means of conduit means C and with means 4, 5 for feeding high purity ammonia and carbon dioxide respectively;
iii) distributing the overall production capacity to apportion from 50 to 95% thereof to the first urea synthesis reactor R and from 5 to 50% to the second urea synthesis reactor ROT, and
iv) reducing the production capacity of the pre-existing reactor R.

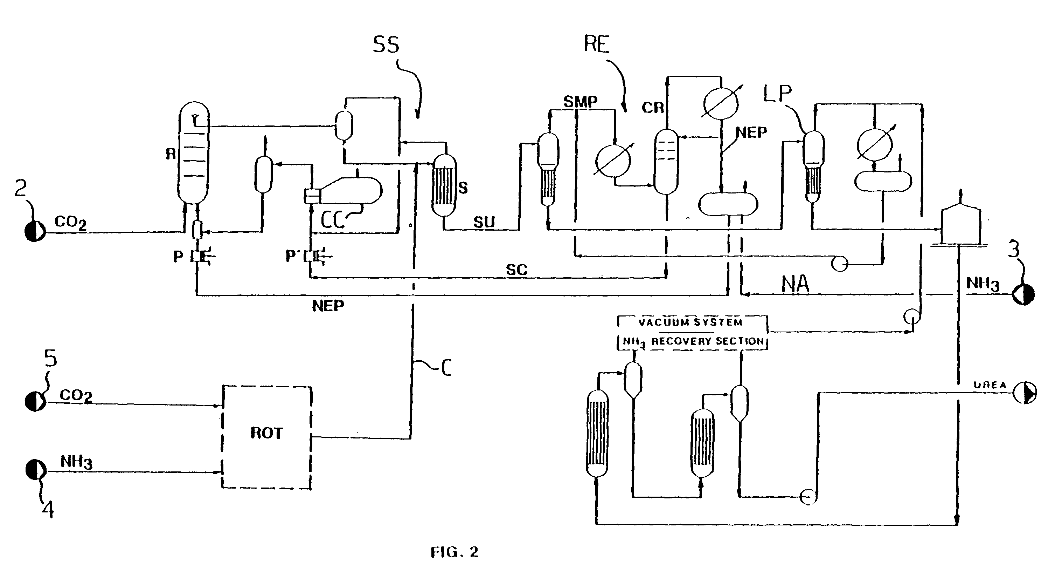

The urea synthesis plant thus obtained is shown in Fig. 2.

According to the present invention, the parallel reactor of the "once through" type ROT, which is connected upstream of the stripper S, may be a reactor of adiabatic type or with partial removal of the reaction heat.

For the purpose of the present invention, the operating pressure and temperature of the "once through" reactor ROT, range from 230 to 270 bar and from 190 to 205°C respectively.

In any event, in the preferred embodiment of this invention, the use of the "once through" reactor ROT with partial reaction heat removal results particularly suitable for the retrofitting of ammonia stripping plants.

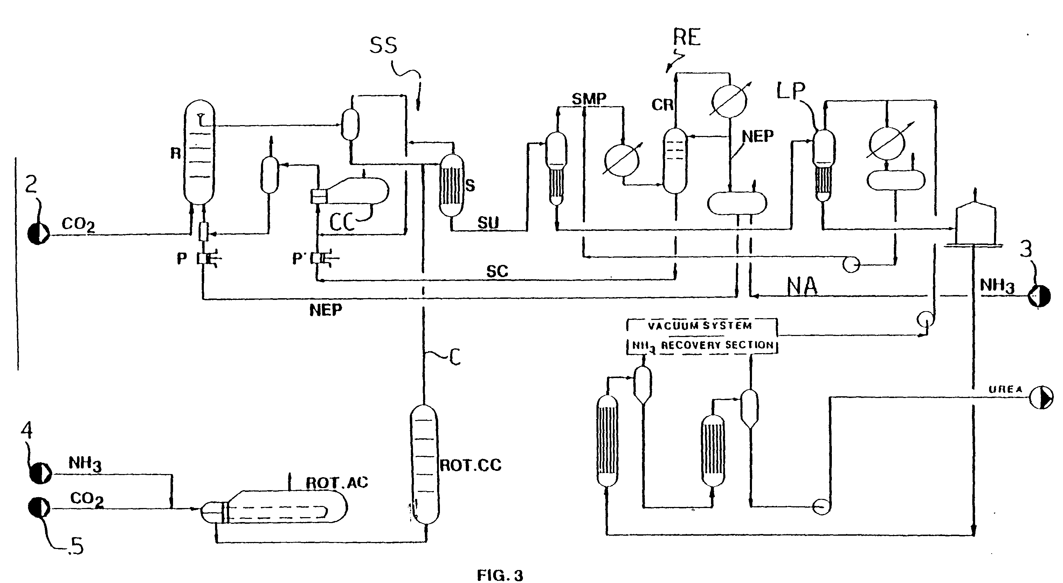

In Fig. 3 is shown a retrofitted urea plant, wherein the parallel reactor ROT comprises two sections:
- a "primary" section ROT.AC with reaction heat removal;
- a "secondary" section ROT.CC of conventional type.

The ROT.AC section preferably comprises a reactor of the so-called "Kettle" type.

In this regard, it should be noted that the use of a "Kettle" type urea synthesis reactor in the method of the present invention, affords the additional advantage of a notable reduction of investment costs, since this kind of reactor may be fed by the pre-existing ammonia pumps, having a maximum delivery pressure higher than the operating pressure of the reactor (260 bar against 240 bar).

According to another aspect of this invention, it is possible to obtain, by distributing the load between the pre-existing reactor R and the added "once through" one ROT, an average conversion yield notably higher than that of the original plant.

Most advantageously, it ensues a greater overloading margin for the key plant equipment, such as the stripper S, the carbamate condenser CC, the medium pressure distillation stage SMP, as well as a considerable "debottlenecking" (i.e. no modification of the key plant equipment) of the plant itself.

In accordance with the present invention, the overall production capacity of the pre-existing plant is distributed so as to apportion from 50 to 95% thereof to the pre-existing urea synthesis reactor R and from 5 to 50% to the added urea synthesis reactor ROT.

Most preferably, the production capacity apportioned to reactor R ranges from 55 to 65%, while the "once through" reactor ROT is apportioned with a corresponding production capacity of from 45 to 35%.

In order to better illustrate the present invention, a non-limitative example of the retrofitting of a pre-existing 1500 MTD urea plant with ammonia stripping is given hereinbelow.

### EXAMPLE

A urea production plant with ammonia stripping having a capacity of 1500 MTD and including a synthesis reactor R operating at 145 bar, a stripping section SS and a recovery section RE including medium pressure (SMP) and low pressure (LP) distillers operating at 18 and 4 bar respectively, was retrofitted so as to have a new capacity of 1500 x 1.5 = 2250 MTD urea.

In order to reduce the thermal load of the pre-existing stripping section SS and recovery section RE (but excluding the vacuum section), the load between the two reactors R, ROT was distributed as follows:

| | |
|---|---|
| - Pre-existing reactor (R) | 1350 MTD urea |
| - added reactor (ROT) | 900 MTD urea |
| - TOTAL | $\overline{\text{2250 MTD urea}}$ |

The operating conditions of the reactors were:
a) Before retrofitting

| Pre-existing reactor R: | |
|---|---|
| capacity | 1500 MTD |
| NH3/CO2 mol | 3.6 |
| H20/CO2 mol | 0.68 |
| Yield | 62% |
| P | 145 bar |
| T | 190°C |

b) After retrofitting

| b.1 pre-existing Reactor R: | |
|---|---|
| Capacity | 1350 MTD |
| NH3/CO2 mol | 3.6 |
| H2O/CO2 mol | 0.75 |
| Yield | 61 % |
| P | 145 bar |
| T | 190°C |

| b.2. "once through" Reactor RC⁻ | |
|---|---|
| Capacity | 900 MTD |
| NH3/CO2 mol | 3.6 |
| H2O/CO2 mol | 0 |
| Yield | 75% |
| P | 242 bar |
| T | 193°C |

The weighed yield of the two reactors operating in parallel was:$\frac{\text{1350 x 61 + 900 x 75}}{\text{2250}} \text{=66.6%}$ i.e. 4.6% higher than the yield of the plant before retrofitting.

This involved a steam consumption decrease of about 100ö130 Kg/MT urea despite the capacity increase of the plant.

Upon retrofitting, the pre-existing stripper S, the recovery section RE including the medium pressure stage SMP and the low pressure distiller LP, and the carbamate condenser CC, did not require any modification in order to be adequate to the increased production capacity.

## Claims

1. A method of retrofitting a pre-existing plant for urea production according to a isobaric ammonia stripping process including a first urea synthesis reactor (R) in fluid communication with an ammonia stripping section (SS) for separating free ammonia and carbamate leaving the reactor from an aqueous urea solution (SU), the free ammonia and carbamate being recycled to said reactor (R), the stripping section (SS) is in turn in fluid communication with a urea recovery section (RE), including a medium pressure distillation stage (SMP), a rectifying column (CR) wherein highly pure ammonia stream (NEP) is separated from a carbamate solution (SC), the pre-existing plant also comprising pump means (P, P') for feeding pure ammonia, obtained by mixing the ammonia stream (NEP) separated from the carbamate solution (SC) with a fresh ammonia stream (NA), and for recycling the carbamate solution (SC) to a carbamate condenser (CC) and to the synthesis reactor (R), respectively, said method including the step of providing a second urea synthesis reactor (ROT) of the once-through type having a higher efficiency yield than said first urea synthesis reactor (R),
**characterized in that** it further comprises the steps of:
a) connecting said second reactor (ROT) to and upstream of said ammonia stripping section (SS) and with means (4, 5) for feeding high purity ammonia and carbon dioxide;
b) distributing overall production capacity to apportion from 50 to 95% of said capacity to said first urea synthesis reactor (R) and from 5 to 50% to said second urea synthesis reactor (ROT);
c) reducing the production capacity of the pre-existing reactor (R).

2. A process according to claim 1, **characterized in that** urea synthesis in the first urea synthesis reactor (R) is carried out at a pressure of about 150 bar and at a temperature of about 190°C.

3. A process according to claim 1, **characterized in that** urea synthesis in the second urea synthesis reactor (ROT) is carried out with a partial removal of reaction heat.

4. A process according to claim 3, **characterized in that** the second urea synthesis reactor (ROT) comprises a primary section (ROT.AC) wherein reaction heat is removed in part and a secondary section (ROT.CC).

5. A process according to claim 4, **characterized in that** said primary section (ROT.AC) comprises a Kettle-type reactor.

6. A process according to claim 1, **characterized in that** urea synthesis in the second urea synthesis reactor (ROT) is carried out at a pressure of about 240 bar and at a temperature of from 190 to 195°C.

## Patentansprüche

1. Verfahren zum Nachrüsten einer bestehenden Anlage zur Produktion von Harnstoff gemäss einem isobaren Ammoniak-Strippprozess mit einem ersten Harnstoffsynthese-Reaktor (R) in Fluidverbindung mit einem Ammoniakstripper-Abschnitt (SS) zum Trennen von freiem Ammoniak und von Carbamaten, welche aus dem Reaktor austreten, von einer wässrigen Harnstofflösung (SU), wobei das freie Ammoniak und das freie Carbamat zum genannten Reaktor (R) rückgeführt werden, der Ammoniakstripper-Abschnitt (SS) steht seinerseits in Fluidverbindung mit einem Harnstoffrückgewinnungsabschnitt (RE), der eine Mitteldruck-Destillationstufe (SMP) und eine Rektifiziersäule (CR), in welche ein Strom von hochreinem Ammoniak (NEP) von der Carbamatlösung (SC) abgetrennt wird, aufweist, die bestehende Anlage weist ebenfalls Pumpenmittel (P, P') auf zum Zuführen von reinem Ammoniak, den man durch Mischen des von der Carbamatlösung (SC) abgetrennten Ammoniakstroms (NEP) mit einem frischen Ammoniakstrom (NA) erhält, und bzw. zum Rückführen der Carbamatlösung (SC) zu einem Carbamatkühler (CC) und zum Synthese-Reaktor (R), umfassend einen zweiten Harnstoffsynthese-Reaktor (ROT) der Durchlauf-Bauart höherer Leistungsausbeute als der erste Harnstoffsynthese-Reaktor (R),
**dadurch gekennzeichnet, dass** ferner folgende Schritte vorgesehen sind:
a) Anschliessen des zweiten Reaktors (ROT) an den Ammoniakstripper-Abschnitt (SS) stromaufwärts davon, wobei Mittel (4, 5) zum Einspeisen von hochreinem Ammoniak und Kohlendioxid vorgesehen sind;
b) Aufteilen der Gesamtproduktionskapazität zu einem Anteil von 50 bis 95% auf den ersten Harnstoffsynthese-Reaktor (R) und von 5 bis 50% auf den zweiten Harnstoffsynthese-Reaktor (ROT).
c) Verringerung der Produktionskapazität des bestehenden Reaktors (R).

2. Verfahren nach Anspruch 1, **dadurch gekennzeichnet, dass** die Harnstoffsynthese in dem ersten Harnstoffsynthese-Reaktor (R) bei einem Druck von 150 bar und einer Temperatur von 190°C durchgeführt wird.

3. Verfahren nach Anspruch 1, **dadurch gekennzeichnet, dass** die Harnstoffsynthese in dem zweiten Harnstoffsynthese-Reaktor (ROT) unter teilweiser Abfuhr der Reaktionswärme durchgeführt wird.

4. Verfahren nach Anspruch 3, **dadurch gekennzeichnet, dass** der zweite Harnstoffsynthese-Reaktor (ROT) einen Primärabschnitt (ROT.AC) aufweist, aus welchem die Reaktionswärme teilweise abgeführt wird, sowie einen Sekundärabschnitt (ROT.CC).

5. Verfahren nach Anspruch 4, **dadurch gekennzeichnet, dass** der besagte Primärabschnitt (ROT.AC) einen Reaktor des Kettle-Typs aufweist.

6. Verfahren nach Anspruch 1, **dadurch gekennzeichnet, dass** die Harnstoffsynthese in dem zweiten Harnstoffsynthese-Reaktor (ROT) bei einem Druck von etwa 240 bar und einer Temperatur im Bereich zwischen 190° und 195°C durchgeführt wird.

## Revendications

1. Méthode pour adapter une usine déjà existante pour la production d'urée, selon un procédé isobarique de stripping d'ammoniac, qui inclut un premier réacteur de synthèse d'urée (R) en communication de fluide avec une section de stripping d'ammoniac (SS) pour séparer l'ammoniac libre et le carbamate sortant du réacteur, d'une solution d'urée aqueuse (SU), l'ammoniac libre et le carbamate étant recyclés vers ledit réacteur (R), la section de stripping (SS) est à son tour en communication de fluide avec une section de récupération d'urée (RE), incluant un étage de distillation à moyenne pression (SMP), une colonne de rectification (CR) où un courant d'ammoniac très pur (NEP) est séparé de la solution de carbamate (SC), l'usine déjà existante comprenant aussi des moyens de pompage (P, P') d'alimentation en ammoniac pur, obtenu en mélangeant le courant d'ammoniac (NEP) séparé de la solution de carbamate (SC) avec un courant d'ammoniac doux (NA), et, respectivement, de recyclage de la solution de carbamate (SC) vers un condensateur de carbamate (CC) et vers le réacteur de synthèse (R ), ladite méthode incluant l'étape de fourniture d'un second réacteur de synthèse d'urée (ROT) du type à passage unique ayant un rendement supérieur à celui dudit premier réacteur de synthèse d'urée (R),
**caractérisée en ce qu'**elle comprend en outre les étapes de:
a) connexion dudit second réacteur (ROT) à ladite section de stripping d'ammoniac (SS) et en amont de celle-ci et aux moyens (4,5) d'alimentation en ammoniac de pureté élevée et en dioxyde de carbone;
b) distribution de la capacité de production globale pour répartir de 50 à 95% de ladite capacité vers ledit premier réacteur de synthèse d'urée (R) et de 5 à 50% vers ledit second réacteur de synthèse d'urée (ROT).
c) réduction de la capacité de production du réacteur déjà existant (R).

2. Procédé selon la revendication 1, **caractérisé en ce que** la synthèse d'urée dans le premier réacteur de synthèse d'urée (R) est réalisée à une pression d'environ 150 bars et à une température d'environ 190°C.

3. Procédé selon la revendication 1, **caractérisé en ce que** la synthèse d'urée dans le second réacteur de synthèse d'urée (ROT) est réalisée avec une élimination partielle de la chaleur de réaction.

4. Procédé selon la revendication 3, **caractérisé en ce que** le second réacteur de synthèse d'urée (ROT) comprend une section primaire (ROT.AC) dans laquelle la chaleur de réaction est éliminée en partie et une section secondaire (ROT.CC).

5. Procédé selon la revendication 4, **caractérisé en ce que** ladite section primaire (ROT.AC) comprend un réacteur de type chaudière.

6. Procédé selon la revendication 1, **caractérisé en ce que** la synthèse d'urée dans le second réacteur de synthèse d'urée (ROT) est réalisée à une pression d'environ 240 bars et à une température de 190 à 195°C.
